# EUROPÄISCHE PATENTSCHRIFT

(11) **EP 2 193 746 B1**
(45) Veröffentlichungstag und Bekanntmachung des Hinweises auf die Patenterteilung: **25.01.2012**
(21) Anmeldenummer: 08170441.3
(22) Anmeldetag: 02.12.2008
(51) Int. Cl.: A61B 5/155

(54) **Gerät zur Gewinnung einer Blutprobe**
Device to obtain a blood sample
Appareil destiné à la production d'un échantillon de sang

(43) Veröffentlichungstag der Anmeldung: 09.06.2010
(73) Patentinhaber: Roche Diagnostics GmbH, 68305 Mannheim (DE); F. Hoffmann-La Roche AG, 4070 Basel (CH)
(72) Erfinder: List, Hans, 64754 Hesseneck-kailbach (DE); Meinecke, Stefan, 68309 Mannheim (DE)
(74) Vertreter: Petirsch, Markus

(56) Entgegenhaltungen:
- EP-A- 1 090 584
- EP-A- 1 852 069
- WO-A-2007/073870
- US-A- 4 924 879
- US-A1- 2005 131 441
- US-A1- 2007 213 682

## Beschreibung

Die Erfindung betrifft ein Gerät zur Gewinnung einer Blutprobe, das eine Lanzette, einen Stechantrieb mit Antriebsfeder und eine Steuereinrichtung zur Steuerung der schnellen Einstich- und Rückzugsbewegung der Lanzette aufweist.

Ein solches Gerät dient vor allem Diabetikern zur regelmäßigen Selbstkontrolle des Blutzuckerspiegels. Dazu wird die Lanzette mit ihrer Spitze in die Haut eines Körperteils, vorzugsweise in die Fingerkuppe, getrieben. Aus der kleinen Wunde tritt dann eine kleine Menge Blut aus, die entweder unmittelbar oder durch eine Kapillare in der Lanzette aufgenommen wird. Anschließend kann diese Blutprobe analysiert werden.

Stand der Technik sind kleine automatische Handgeräte mit integrierter Messeinheit und als Mikronadel mit Kapillare ausgebildete Lanzette, die als Einwegartikel (Disposables) ausgebildet sind. Dabei ist ein Vorrat von Einweg-Lanzetten in einem Magazin gespeichert. Der Austausch einer benutzten Lanzette gegen eine neue erfolgt automatisch, so dass der Anwender nicht mit Einzelteilen hantieren muss.

WO 2007/073870 beschreibt ein Microsampler-Stechsystem, dessen Stechantrieb eine Schraubenfeder umfasst, die eine Schubstange nach vorne treibt. Der Kopf der Schubstange ist an die Lanzette ankoppelbar. Die Bewegung der Lanzette in axialer Richtung folgt einem vorgegebenen Bewegungsprofil und umfasst eine Vortriebsphase, bei der die Lanzette bis zu einer vorgegebenen maximalen Einstichtiefe eingestochen wird, und eine unmittelbar daran anschließende Rückzugsphase, bei der die Lanzette bis zu einer vorgegebenen Reststichtiefe teilweise wieder zurückgezogen wird. Es wurde nämlich festgestellt, dass für eine optimale Probengewinnung und geringstmöglichen Schmerz die Reststichtiefe von großer Bedeutung ist. Am Ende der Vortriebsphase kontaktiert die Schubstange einen Anschlag. Dabei ist die Antriebsfeder überstreckt, so dass die Schubstange mit der angekoppelten Lanzette während der anschließenden Rückzugsphase von der Antriebsfeder auch wieder zurückgezogen wird. Alternativ umfasst der Stechantrieb eine Torsionsfeder, einen Spannrotor und einen von der gespannten Feder angetriebenen Antriebsrotor. Zum Umsetzen der Drehbewegung des Antriebsrotors in eine lineare Einstich-/und Rückführbewegung eines Stechelements dient eine Steuereinrichtung in Form einer Kurvensteuerung. Eine Lanzetten-Steuerkurve ist als Nut in dem Antriebsrotor ausgebildet und wird von einem Steuerkurvenreiter abgefahren, der mit dem Lanzettenhalter verbunden ist.

US 6 206 901 B1 beschreibt eine Blutentnahmevorrichtung mit einer Lanzette, die über einen Stößel und eine Antriebsfeder angetrieben wird. Dabei ist die Lanzette auf einem Teil ihrer Vortriebsphase von der Antriebsfeder entkoppelt. Um die Lanzette nach dem Einstich wieder aus der Wunde zurückzuführen, ist eine zweite Feder vorgesehen. Diese Rückführfeder wird erst während der Vortriebsphase gespannt, indem kinetische Energie der Lanzette in der Feder gespeichert und nach Erreichen der maximalen Spannung der Rückführfeder von dieser an die Lanzette zurückgegeben wird. Nachteilig ist, dass die Lanzette durch die Rückführfeder vorzeitig abgebremst wird. Dadurch ist der Eintritt der Lanzette in die Haut des Patienten langsamer und schmerzhafter.

Auch EP 0 036 443 B1 beschreibt eine Lanzettenvorrichtung, bei der die Lanzette während eines Teils der Stechbewegung von der Antriebsfeder entkoppelt ist. Die Rückführung der Lanzette nach dem Einstich erfolgt durch eine zweite Feder, die durch die Lanzette während deren Vortriebsbewegung gespannt wird.

Stechantriebe mit einer Antriebsfeder, deren Federkraft linear in Einstichrichtung direkt auf die Lanzette übertragen wird, sind konstruktiv einfach und damit ebenso zuverlässig wie kostengünstig und überdies platzsparend. Sie ermöglichen insbesondere den Einbau in ein schmales Handgerät, das vom Benutzer bequem und leibt handhabbar ist. Um einen möglichst schnellen und schmerzarmen Einstich zu gewährleisten, werden ziemlich starke Federn eingesetzt. Dabei besteht die Gefahr, dass durch ein Nachschwingen die Nadel mehrfach einsticht. Bei dem Blutentnahmesystem gemäß EP 1 852 069 A1 ist deshalb eine Schwingungskontrolleinrichtung vorgesehen, die auf das schwingungsfähige System aus Lanzette und Antriebsfeder so einwirkt, dass ein mehrfaches Einstechen verhindert wird. Beschrieben werden drei Arten der Einwirkung auf das Schwingungsverhalten der Lanzette: Zum einen kann ein verschiebbarer Anschlag die Schwingungsbewegung der Lanzette in Einstichrichtung begrenzen. Als zweite Möglichkeit kann eine Dämpfungseinrichtung, beispielsweise in Form eines hydraulischen oder pneumatischen Dämpfers, vorgesehen werden. Als weitere Möglichkeit kann eine Ortsverschiebung der Antriebsfeder vorgesehen sein, durch die der Nullpunkt der Schwingung der Antriebsfeder so verschoben wird, dass auch bei maximaler Auslenkung der Antriebsfeder ein nochmaliges Einstechen der Lanzette ausgeschlossen ist. Die Schwingung selbst wird dabei nicht beeinflusst.

Bei Microsampler-Stechgeräten, die einen Vorrat von Einmal-Lanzetten enthalten, muss neben dem eigentlichen Stechantrieb, der kurze schnelle Vorund Rückbewegungen ausführt, zusätzlich auch ein Mechanismus vorgesehen werden, der vergleichsweise langsame, dafür lange Betätigungshübe bereitstellt, um die magazinierten Lanzetten an den Stechantrieb anzudocken und nach einmaliger Benutzung wieder frei zu geben.

Stechantriebe, deren Steuereinrichtung mit einer Steuerkurve arbeitet, sind prinzipiell gut für abwechselnd schnelle und langsame Bewegungsphasen geeignet, haben aber bei großen Hüben den Nachteil, dass sie vergleichsweise groß sind, da anderenfalls die Steigungswinkel der Steuerkurve bis zur Selbsthemmung anwachsen. EP 1 090 584 beschreibt einen solchen Stechantrieb. Dem kann dadurch begegnet werden, dass ein schneller kurzhubiger Stechantrieb, der die Lanzette antreibt, auf einen Schiebeschlitten montiert wird, der die großen langsamen Bewegungen für die Ineingriff- und Außereingriffnahme der Lanzette ausführt. Dieser Schiebeschlitten kann gleichzeitig auch dazu benutzt werden, den Stechantrieb zu spannen, beispielsweise indem der Stößel, der die Lanzette nach vorne treiben soll, gegen einen ortsfest im Gehäuse montierten Anschlag gefahren wird, bis die Antriebsfeder gespannt und im Auslösemechanismus verrastet ist. Solche so genannten "ballistischen" Antriebe bieten jedoch bisher keine Möglichkeit, nach dem Erreichen der maximalen Einstichtiefe schnell und ohne Nachschwingen eine definierte Position einzunehmen, die der Reststichtiefe entspricht. Dies ist aber sowohl für die Gewinnung einer ausreichenden Blutprobe wie auch die angestrebte Schmerzarmut wichtig. Wird der Stechantrieb einfach mit einem mechanischen Anschlag ausgerüstet, der die maximale Einstichtiefe begrenzt, so kommt es im Moment des tiefsten Einstichs zu einem harten Aufprall, was nicht nur ein irritierendes Geräusch verursacht, sondern auch eine Schwingung der Nadel auslöst, die zu erhöhtem Schmerzempfinden führt.

Der vorliegenden Erfindung liegt somit das technische Problem zugrunde, einen konstruktiv einfachen Stechantrieb bereitzustellen, der mit einer einzigen, in Richtung der Bewegungsachse der Lanzette wirkenden Antriebsfeder auskommt und dennoch einen schwingungsfreien Einstich erlaubt. Insbesondere soll der Stechantrieb auch dazu geeignet sein, im Rahmen eines Schiebemechanismus, der zum langsamen vollständigen Rückzug der Lanzette, zum Andocken und Freigeben magazinierter Einmal-Lanzetten und/oder zum Spannen der Antriebsfeder dient, eingesetzt zu werden.

Gelöst wird die gestellte Aufgabe gemäß dem kennzeichnenden Teil des ersten Patentanspruchs durch eine beweglich gelagerte Schwungmasse und ein Koppelglied, das die Schwungmasse an die Treibstange bzw. die Lanzette ankoppelt. Durch den Vortrieb der Lanzette wird die Schwungmasse in Bewegung versetzt. Dabei wird ein Teil der kinetischen Energie der Treibstange und der Lanzette zur Beschleunigung der Schwungmasse eingesetzt. Hat die Lanzette den Umkehrpunkt erreicht, der die maximale Einstichtiefe definiert, bewirkt die in der Schwungmasse gespeicherte Bewegungsenergie die Rückzugsbewegung der Lanzette. Dabei wird die aufgenommene kinetische Energie wieder auf die Treibstange und die angekoppelte Lanzette zurück übertragen. Durch die angekoppelte Schwungmasse lässt sich ein sanfter Übergang von der Vorwärts- in die Rückzugsbewegung der Lanzette realisieren und damit ein besonders schwingungsfreier schmerzarmer Einstich.

Bei einer bevorzugten Ausführung der Erfindung ist ein Koppelgetriebe vorgesehen, welches wenigstens einen, entlang einer linearen Bewegungsbahn bewegbaren Schwungkörper umfasst sowie eine Koppelstange, deren eines Ende mit der Treibstange verbunden ist und deren anderes Ende an dem Schwungkörper beweglich gelagert ist. Das Koppelgetriebe überträgt die Vorwärtsbewegung der Treibstange mit der angekoppelten Lanzette auf den Schwungkörper, der dadurch in eine translatorische Bewegung versetzt wird. Das Koppelgetriebe bewirkt nicht den Vortrieb der Lanzette, sondern läuft passiv mit. Die Kraft der sich entspannenden Feder wird nicht nur zum Antrieb der Lanzette benutzt, sondern gleichzeitig auch zur Beschleunigung des Schwungkörpers. Durchfährt die Lanzette den Umkehrpunkt, welcher der maximalen Einstichtiefe entspricht, so sorgt die träge Masse des Schwungkörpers dafür, dass er weiter in Bewegung bleibt. Diese fortgesetzte translatorische Bewegung überträgt sich durch die Koppelstange auf die Treibstange und leitet dadurch die Rückzugsbewegung der Lanzette ein. Dabei wird die in dem Schwungkörper gespeicherte Bewegungsenergie in kinetische Energie der Treibstange und der Lanzette umgesetzt. Das Koppelgetriebe steuert und begrenzt zudem die Einstichtiefe sehr präzise.

Vorzugsweise verläuft die Bewegungsbahn des Schwungkörpers im Wesentlichen quer zur Stichachse der Lanzette. Bevorzugt ist die Bewegungsbahn eine Gerade. Dadurch, dass sich der Schwungkörper im Wesentlichen quer zur Stichachse bewegt, ist der Weg der Treibstange und damit der Lanzette in Vortriebsrichtung begrenzt. Ab dem Umkehrpunkt führt jede weitere Bewegung des Schwungkörpers zwangsweise dazu, dass sich die Treibstange und die Lanzette wieder nach unten bewegen müssen. Diese Kinematik lässt sich dazu benutzen, der Stichbewegung der Lanzette einen Übergang von der Vorwärts- in die Rückwärtsbewegung mit vorgegebenem Geschwindigkeitsprofil aufzuprägen. Das Geschwindigkeitsprofil, mit dem die Lanzette am Ende der Vorwärtsbewegung abgebremst und sofort anschließend wieder zurückgezogen wird, hängt entscheidend vom Verhältnis zwischen der trägen Masse des Schwungkörpers und der Federkraft der Antriebsfeder ab. Eine Feinabstimmung des Systems ist durch eine Veränderung der Masse des Schwungkörpers möglich.

Verläuft die Bewegungsbahn des Schwungkörpers nicht rechtwinklig zur Stichachse, dann führt der Lagerpunkt der Koppelstange auf dem Schwungkörper eine Bewegung aus, die einen Anteil parallel zur Stichrichtung hat. Der parallel zur Stichachse wirkende Bewegungsanteil addiert sich dann zur Vor- bzw. Rückbewegung der Lanzette. Damit lässt sich das Verhältnis der kinetischen Energien, die von der Treibstange auf den Schwungkörper und umgekehrt übertragen werden, variieren. Zweckmäßig beträgt der Winkel, unter dem die Bewegungsbahn des Schwungkörpers schräg gegenüber der Stichachse verläuft, zwischen 5 und 45 Grad.

Bewegt sich der Schwungkörper nicht entlang einer Geraden, sondern ist dessen Bewegungsbahn gekrümmt, so werden weitere Freiheitsgrade geschaffen, um das Geschwindigkeits- und/oder Beschleunigungsprofil der Lanzette speziell im Bereich der maximalen Einstichtiefe optimal zu gestalten.

Bei einer weiteren bevorzugten Ausführung der Erfindung ist ein Kurbeltrieb vorgesehen, welcher wenigstens ein drehbar gelagertes Schwungrad umfasst sowie eine Kurbelstange, deren eines Ende mit der Treibstange für die Lanzette verbunden und deren anderes Ende auf dem Schwungrad exzentrisch gelagert ist. Der Kurbeltrieb setzt die oszillierende translatorische Bewegung der Lanzette in eine rotatorische Bewegung des Schwungrads um, vergleichbar einer Kurbelwelle mit Pleuel bei einer Kolbenmaschine. Der Kurbeltrieb bewirkt nicht den Vortrieb der Lanzette, sondern steuert und begrenzt die Einstich- und Rückzugsbewegung der Lanzette. Wird die Lanzette durch die Spannkraft der Antriebsfeder nach vorne getrieben, so wird dadurch das Schwungrad in Rotation versetzt. Der obere Totpunkt des Kurbeltriebs definiert die maximale Einstichtiefe der Lanzette. Ist der obere Totpunkt überschritten, so bewirkt die in dem Schwungrad gespeicherte Rotationsenergie die Umkehr der Bewegungsrichtung der Lanzette und schließlich deren schneller Rückzug bis zur vorgegebenen Reststichtiefe. Das Geschwindigkeitsprofil, mit dem die Lanzette abgebremst und wieder zurückgezogen wird, hängt entscheidend vom Verhältnis zwischen der trägen Masse des Schwungrads und der Federkraft der Antriebsfeder ab. Eine Feinabstimmung des Systems ist durch eine Veränderung der Masse des Schwungrads auf einfache Weise möglich.

Gegenüber Stechantrieben, bei denen der Vortrieb der Lanzette durch einen harten Anschlag begrenzt wird oder eine zweite Feder die Rückzugsbewegung übernimmt, wird bei der erfindungsgemäßen Konstruktion der obere Totpunkt durch die Umsetzung einer Kreisbewegung in eine translatorische Bewegung angesteuert, was automatisch zu einer nichtlinearen Charakteristik, d.h. zu einem weichen, aber präzise kontrollierten Bewegungsablauf führt.

Vorzugsweise ist das Schwungrad quer zur Stichachse der Lanzette drehbar gelagert, so dass die Drehachse des Schwungrads und die Stichachse der Lanzette senkrecht zueinander stehen. An Stelle eines solchen gewöhnlichen Kurbeltriebs kann aber auch eine Anordnung verwendet werden, bei der die Drehachse des Schwungrads parallel zur Stichachse der Lanzette verläuft. Insbesondere bei sehr leichter Ausführung der Treibstange kann dadurch Bauraum gespart werden.

Der vorgeschlagene Kurbeltrieb, bestehend aus Schwungrad und mit der Lanzette verbundener Kurbelstange, erzwingt ein Umkehren der Bewegungsrichtung der Lanzette im oberen Totpunkt unabhängig davon, ob in diesem Moment die Antriebsfeder schon vollständig entspannt ist oder diese gar schon überstreckt ist. Bevorzugt ist allerdings eine Ausführung, bei der die Rückzugsbewegung entgegen der Wirkung der noch nicht völlig entspannten Antriebsfeder erfolgt. Dabei wird die Federkraft zwar etwas früher in Rotationsenergie umgesetzt, jedoch wird durch die Rückzugsbewegung der Lanzette entgegen einer gewissen Rest-Spannkraft der Druckfeder die Gefahr einer Schwingung, die im Bereich der maximalen Einstichtiefe besonders störend wäre, zuverlässig vermieden.

Der Einsatz eines Kurbeltriebs in einem Stechantrieb ist an sich bereits aus US 4 924 879 bekannt. Dort ist die Antriebsfeder als Spiralfeder ausgebildet und wirkt direkt auf ein Kurbelrad, so dass dieses rotiert. Die Drehung des Kurbelrads wird dann durch eine Schubstange in eine lineare Bewegung der Lanzette umgesetzt. Der Kraftfluss in der Vortriebsphase ist also genau umgekehrt wie bei der erfindungsgemäßen Konstruktion, bei der zunächst die Antriebsfeder die Lanzette in Einstichrichtung vorwärts treibt und der Kurbeltrieb passiv mitläuft.

Der Kurbeltrieb ist vorzugsweise seitlich versetzt zur Stichachse der Lanzette angeordnet. Das exzentrisch, vorzugsweise im Bereich des äußeren Randes des Schwungrads angeordnete Drehlager der Kurbelstange bewegt sich damit entlang eines Kreisbogens, so dass sich der seitliche Abstand zur Bewegungsachse der Lanzette während der Vor- und Rückbewegung der Lanzette ändert. Je größer der Abstand und damit der Winkel ist, unter dem die Kurbelstange an der Treibstange angreift, desto langsamer dreht sich das Schwungrad. Dies führt dazu, dass zu Beginn der Vortriebsphase das Schwungrad nur mäßig beschleunigt wird, kurz vor Erreichen des oberen Totpunkts jedoch sehr stark. Nach dem Umkehrpunkt der Lanzette sorgt die träge Masse des Schwungrads dafür, dass es sich weiterdreht. Der angekoppelte Kurbeltrieb mit Schwungrad sorgt also zuerst für eine starke Abbremsung der Lanzette kurz vor Erreichen der maximalen Einstichtiefe und bewirkt anschließend den schnellen Rückzug bis zur Reststichtiefe. Gleichzeitig definiert und begrenzt der Kurbeltrieb die Einstichtiefe.

In vorteilhafter Weiterbildung der Erfindung ist die Antriebsfeder eine Schraubenfeder, vorzugsweise eine koaxial um die Treibstange angeordnete Druckfeder, ist auf der Treibstange ein Druckstück vorgesehen, gegen das die Druckfeder drückt, und ist die Kurbelstange des Kurbeltriebs über das Druckstück mit der Treibstange verbunden. Das Druckstück hat zwei Funktionen: Zum einen bildet es eine Anlagefläche für die Druckfeder und überträgt deren Federkraft auf die Treibstange, zum anderen bildet es einen Lagerblock für das obere Drehlager der Kurbelstange. Zweckmäßig wird man das Druckstück so bemessen, dass es seitlich über die Druckfeder hinaus auskragt, so dass die Kurbelstange in allen Bewegungsphasen ausreichenden Abstand zur Druckfeder hat. Damit kann die Kurbelstange relativ kurz ausgeführt werden, so dass der Stechantrieb kompakt bleibt.

Nach der Rückzugsphase sollte die Lanzette möglichst schnell in der Position fixiert werden, die der vorgegebenen Reststichtiefe entspricht. Am einfachsten geschieht dies durch ein Anschlagelement, das sich mit dem Schwungrad mitdreht und bei einer vorgegebenen Winkelstellung auf ein festes Gegenanschlagelement trifft. Damit wird die Drehbewegung des Schwungrads am Ende der Rückzugsphase gestoppt und die Reststichtiefe definiert.

Damit sich nach Erreichen der Reststichtiefe die Lanzette auch nicht mehr nach vorne bewegen kann, was ja zu einem erneuten schmerzhaften Einstich führen würde, ist es zweckmäßig, das Anschlagelement an dem Gegenanschlagelement festzulegen. Bevorzugt geschieht dies durch Magnetkraft, so dass das Anschlagelement an dem Gegenanschlagelement magnetisch haftet. Die Magnetkraft sollte ausreichend groß sein, um den gesamten Stechantrieb festzulegen.

Bei einem seitlichen Versatz zwischen Drehachse des Schwungrads und Stichachse der Lanzette wirken unweigerlich Querkräfte auf die Treibstange. Dadurch kann es zu störenden Reibungen in den Führungslagem kommen. Deshalb ist es vorteilhaft, den Kurbeltrieb symmetrisch aufzubauen, also zwei Schwungräder einander gegenüberliegend zu beiden Seiten der Treibstange anzuordnen. Das Druckstück auf der Treibstange kann dann ein Querjoch umfassen, so dass die beiden gegenüberliegenden Enden des Querjochs durch je eine Kurbelstange mit den Schwungrädern verbindbar sind. Die von den Kurbelstangen über das Druckstück auf die Treibstange übertragenen Seitenkräfte heben sich bei dieser symmetrischen Anordnung auf.

Die beiden Kurbeltriebe und insbesondere deren Schwungräder sind vorzugsweise miteinander zu synchronisieren, beispielsweise dadurch, dass die beiden Schwungräder an ihren Stirnseiten ineinander greifende Verzahnungen aufweisen. Um ein Verklemmen der beiden Kurbeltriebe zu verhindern, ist es zweckmäßig, die beiden spiegelbildlichen Kurbelstangen über ein weiteres Drehgelenk mit der Treibstange zu verbinden. Vorzugsweise geschieht dies durch ein Querjoch, das schwenkbeweglich an der Treibstange gelagert ist. Da hier nur sehr kleine Schwenkbewegungen auftreten, kann die erforderliche Beweglichkeit beispielsweise durch ein Filmscharnier hergestellt werden, wodurch der Fertigungsaufwand äußerst gering gehalten wird.

Besonders bevorzugt wird eine Ausführung, bei welcher der Stechantrieb einschließlich Druckfeder und der Kurbeltrieb umfassend mindestens ein Schwungrad gemeinsam auf einem Schiebeschlitten angeordnet sind, der parallel zur Bewegungsachse der Lanzette verschieblich ist. Wird nämlich der Stechantrieb nach Erreichen der Reststichtiefe blockiert, so wird die Treibstange zu einem gegenüber dem Schiebeschlitten nicht mehr beweglichen Bauteil. Der Schiebeschlitten kann dann die langhubigen und langsamen Bewegungen ausführen, die zum vollständigen Rückzug der Lanzette dienen und über die blockierte Treibstange auf die Lanzette übertragen werden. Vorteilhaft kann der Schiebeschlitten auch zum Spannen des Stechantriebs benutzt werden, indem ein Spannanschlag an einem Schwungrad gegen einen ortsfesten Gegenanschlag gefahren wird.

Ein Ausführungsbeispiel der Erfindung wird nachstehend anhand der beigefügten Zeichnungen beschrieben. Es zeigen:
- Figur 1a: ein erstes Gerät zur Gewinnung einer Blutprobe, mit einem Kurbel- trieb und einem Schwungrad während der Vortriebsphase der Lanzette, in einem Prinzipbild;
- Figur 1b: das Gerät von Fig. 1a bei maximaler Einstichtiefe;
- Figur 1c: das Gerät nach Fig. 1a bei Reststichtiefe;
- Figur 2a: ein zweites Gerät zur Gewinnung einer Blutprobe, mit zwei sym- metrischen Kurbeltrieben und zwei Schwungrädern, in einer ver- einfachten perspektivischen Ansicht von oben;
- Figur 2b: das Gerät nach Fig. 2a, von unten gesehen;
- Figur 3a: ein drittes Gerät zur Gewinnung einer Blutprobe, mit einem Kop- pelgetriebe und Schwungkörper während der Vortriebsphase der Lanzette, in einem Prinzipbild;
- Figur 3b: das Gerät von Fig. 3a bei maximaler Einstichtiefe;
- Figur 3c: das Gerät nach Fig. 3a bei Reststichtiefe;
- Figur 4a: ein viertes Gerät zur Gewinnung einer Blutprobe, mit einem Kop- pelgetriebe und schräger Bewegungsbahn des Schwungkörpers während der Vortriebsphase der Lanzette, in einem Prinzipbild;
- Figur 4b: das Gerät von Fig. 4a bei maximaler Einstichtiefe;
- Figur 4c: das Gerät nach Fig. 4a bei Reststichtiefe.

In den Abbildungen sind gleiche Teile jeweils mit gleichen Bezugszeichen versehen.

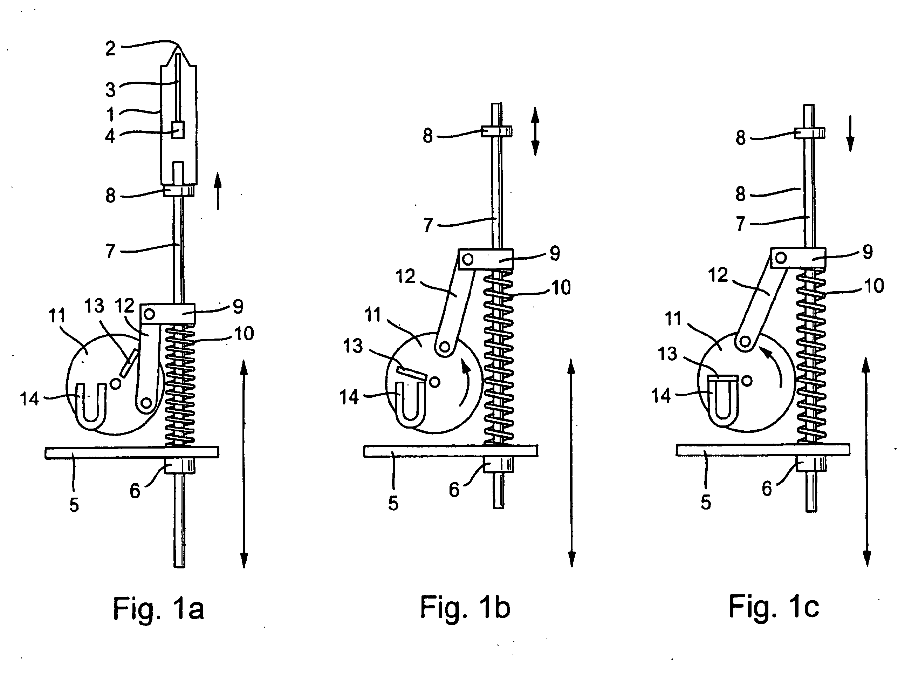

Die Prinzipbilder der Figuren 1 a, b und c zeigen nur die wesentlichen Elemente der Antriebsmechanik eines Geräts zur Gewinnung einer Blutprobe mittels einer Lanzette 1, die in die Haut eines Körperteils eingestochen und sofort anschließend wieder ein Stück herausgezogen wird, um das aus der Wunde austretende Blut aufzunehmen. Die Lanzette 1 ist als Einwegartikel (Disposable) ausgebildet und umfasst eine Stechspitze 2, einen Kapillarkanal 3 und einen kleinen Blutsammelraum 4.

Der Stechantrieb ist auf einen Schiebeschlitten 5 montiert, der langsame langhubige Bewegungen - angedeutet durch den langen Doppelpfeil - ausführen kann, um die Lanzette 1 nach einmaligem Gebrauch gegen ein neues Exemplar auszutauschen.

Der Schiebeschlitten 5 trägt ein Gleitlager 6, in dem eine runde Treibstange 7 in axialer Richtung verschieblich so gelagert ist, dass die Treibstange 7 den Schiebeschlitten 5 durchsetzt. An ihrem oberen Ende weist die Treibstange 7 eine Kupplung 8 auf, mittels der die Lanzette 1 an die Treibstange 7 lösbar angekoppelt ist. In einigem Abstand unterhalb der Kupplung 8 ist an die Treibstange 7 ein Druckstück 9 fest anmontiert. Um die Treibstange 7 herum ist koaxial eine Druckfeder 10 angeordnet, deren unteres Ende am Schiebeschlitten 5 anliegt. Die Druckfeder 10 ist eine zylindrische Schraubenfeder aus Stahl mit linearer Federcharakteristik. Mit ihrem oberen Ende drückt die Druckfeder 10 gegen die Unterseite des Druckstücks 9, so dass sie die Treibstange 7 und die angekoppelte Lanzette 1 in Richtung des kleinen Pfeils nach vorne treibt. Die Bewegungsachse der Treibstange 7 mit der Lanzette 1 und die Bewegungsrichtung des Schiebeschlittens 5 sind zueinander parallel. In Fig. 1a ist die Druckfeder 10 bereits nicht mehr vollständig gespannt, die Lanzette befindet sich also in der schnellen Vortriebsphase.

Seitlich neben der Treibstange 7 ist ein Schwungrad 11 quer zur Bewegungsachse der Lanzette 1 drehbar gelagert. Eine Kurbelstange 12 ist mit ihrem einen Ende im Bereich des Randes des Schwungrads 11 drehbar gelagert, während das andere Ende der Kurbelstange 12 auf dem Druckstück 9 drehbar gelagert ist. Das Druckstück 9 kragt seitlich so weit aus, dass die Kurbelstange 12 nicht in Kontakt mit den Windungen der Druckfeder 10 gerät. Das Schwungrad 11 trägt ein mitbewegtes Anschlagelement 13, das in Fig. 1a noch relativ weit entfernt ist von dem zugehörigen Gegenanschlag 14, der auf dem Schiebeschlitten 5 fest montiert ist.

Schwungrad 11 und Kurbelstange 12 bilden einen passiv mitlaufenden Kurbeltrieb, der die translatorische Bewegung der Treibstange 7 in eine Drehbewegung des Schwungrads 11 umsetzt. Dabei wird ein Teil der kinetischen Energie der Treibstange 7 und der angekoppelten Lanzette 1 in Rotationsenergie des Schwungrads 11 umgesetzt.

In Fig. 1b hat die Lanzette 1 die maximale Einstichtiefe erreicht. Der Kurbeltrieb befindet sich im oberen Totpunkt. Die Druckfeder 10 ist maximal ausgelenkt. Das Schwungrad 11 hat sich um etwa 90° im Gegenuhrzeigersinn gedreht. Das Anschlagelement 13 steht kurz vor dem Gegenanschlag 14.

In Fig. 1c ist der obere Totpunkt überwunden. Die in dem Schwungrad 11 gespeicherte Rotationsenergie hat die Rückzugsbewegung der Lanzette 1 bewirkt, und zwar gegen die Spannkraft der Druckfeder 10, die schon wieder etwas kürzer geworden ist. Rotationsenergie des Schwungrads 11 wurde also zurück übertragen auf die Treibstange 7 und in potentielle Energie der Druckfeder 10 umgesetzt. Die Drehbewegung des Schwungrads 11 wurde am Ende gestoppt, indem das Anschlagelement 13 am Gegenanschlag 14 angekommen ist. Dadurch wurde auch die lineare Bewegung der Treibstange 7 und der angekoppelten Lanzette 1 zunächst abgebremst und schließlich in einer Position gestoppt, die der vorgegebenen Reststichtiefe entspricht.

Das Gegenanschlagelement 14 ist hier als Permanentmagnet ausgebildet, dessen Pole durch das Anschlagelement 13, das als magnetischer Anker ausgebildet ist, geschlossen werden. In der Endstellung werden also Anschlagelement 13 und Gegenanschlag 14 durch Magnetkraft aufeinander gehalten. Damit ist der Stechantrieb festgelegt. Ein Zurückschwingen der Lanzette 1 mit der Gefahr eines nochmaligen Einstiches ist dadurch ausgeschlossen.

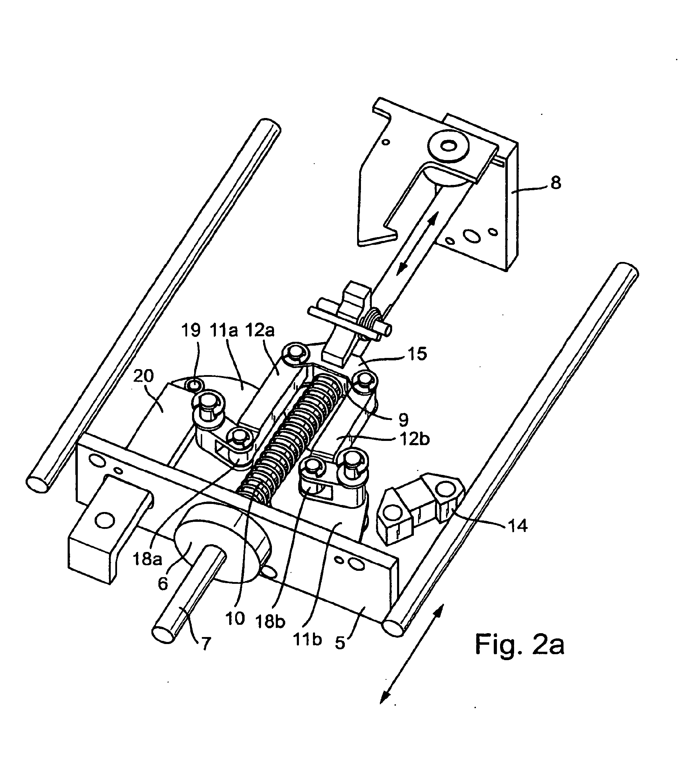

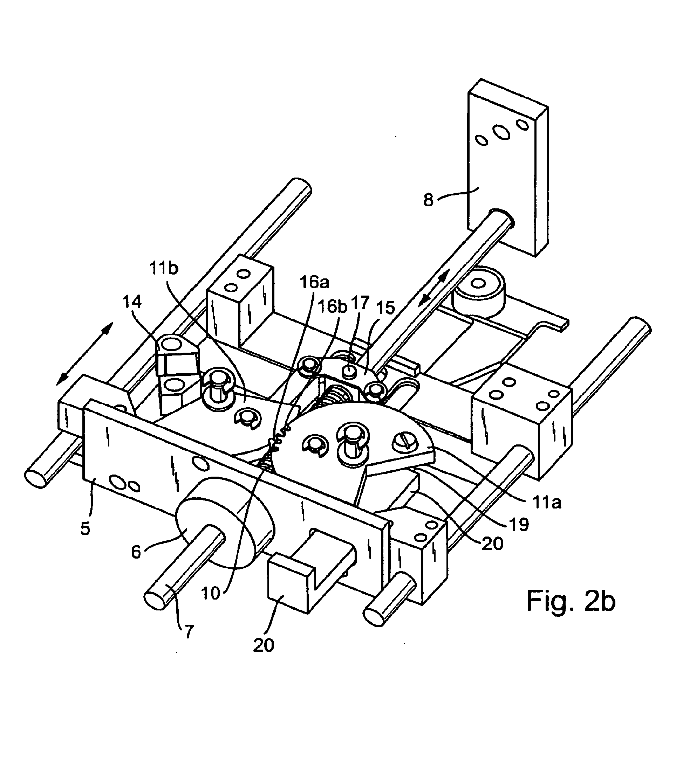

Die konstruktive Ausgestaltung des Stechantriebs gemäß den Figuren 2a und 2b umfasst zwei Schwungräder 11a und 11b, die symmetrisch zu beiden Seiten der Treibstange 7 angeordnet sind. Das Druckstück 9 trägt ein Querjoch 15, dessen beiden gegenüberliegenden Enden durch je eine Kurbelstange 8a und 8b mit den Schwungrädern 11a bzw. 11 b verbunden sind. Die Schwungräder 11a, 11b weisen an ihren Stirnseiten Verzahnungen 16a, 16b auf, die ineinander greifen. Dadurch sind die beiden Schwungräder 11a, 11b miteinander zwangssynchronisiert. Das Querjoch 15 ist mittels eines Drehgelenks 17 schwenkbeweglich gelagert. Dies gibt dem mechanischen System den nötigen Freiheitsgrad, um auch bei einer eventuellen Schiefstellung der Treibstange 7 hemmungsfrei zu laufen.

In Fig. 2a ist zu sehen, dass die Schwungräder 11a, 11b in der Art einfacher Kurbelwellen ausgebildet sind, mit einer einzigen Kurbelwange und je einem Kurbelzapfen 18a bzw. 18b, auf denen die Kurbelstangen 12a, 12b wie Pleuel gelagert sind.

Befinden sich die Schwungräder 11a, 11 b in ihrer Endstellung, die der Reststichtiefe entspricht, sind diese durch das Aneinanderhaften von Anschlagelement 13 und Gegenanschlag 14 (vgl. Fig. 1c) blockiert. Damit ist auch die Treibstange 7 starr auf dem Schiebeschlitten 5 festgelegt. Eine Verschiebung des Schiebeschlittens 5 in Richtung der beiden Pfeile nach unten überträgt sich also auf die Treibstange 7. Dadurch kann nicht nur die langsame Rückzugsbewegung der Lanzette über die Reststichtiefe hinaus ausgeführt werden, sondern auch die langhubigen Bewegungen für das An- und Abkoppeln der Lanzette.

Auf dem in Fig. 2a linken Schwungrad 11a ist am Rand ein Spannanschlag 19 vorgesehen. Beim Verfahren des Schiebeschlittens 5 entgegen der Vortriebsrichtung der Lanzette 1 trifft der Spannanschlag 19 auf einen Spanngegenanschlag 20, der außerhalb des Schiebeschlittens 5 ortsfest im Gerätegehäuse montiert ist. Dadurch wird das Schwungrad 11a zurückgedreht, wobei die Haftkraft zwischen Anschlagelement 13 und Gegenanschlag 14 überwunden wird. Auf diese Weise lässt sich die Druckfeder 10 spannen.

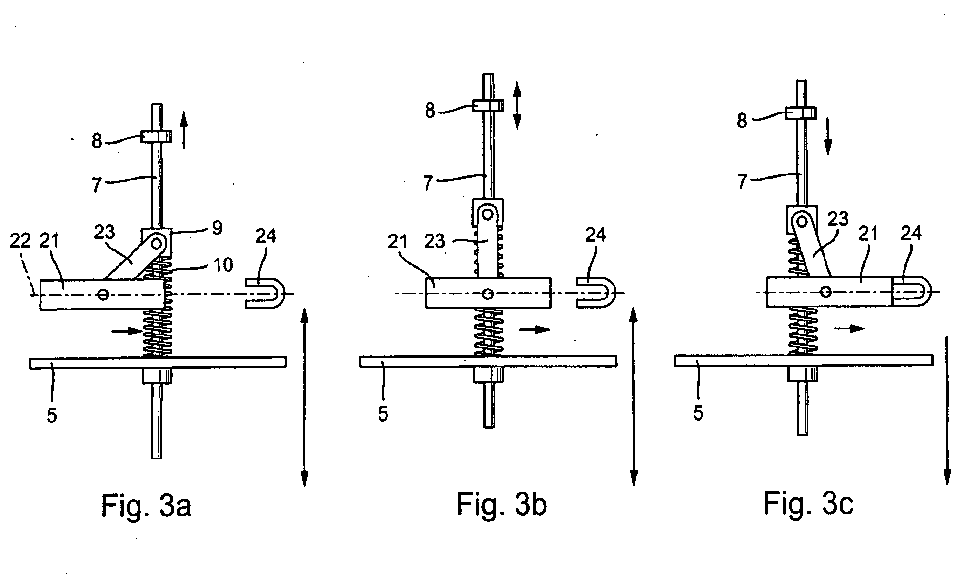

Die Prinzipbilder der Figuren 3a, b und c zeigen eine alternative Ausführung der Antriebsmechanik.

Die Treibstange 7 trägt ein Druckstück 9. Eine Druckfeder 10 drückt gegen die Unterseite des Druckstücks 9 so, dass die Treibstange 7 in Richtung des kleinen Pfeils nach vorne getrieben wird. Der Stechantrieb sitzt auf einem Schiebeschlitten 5, der in Richtung des großen Pfeils relativ langsam verschieblich ist.

Ein quaderförmiger Schwungkörper 21 ist entlang einer geraden Bewegungsbahn 22 verschieblich gelagert. Eine Koppelstange 23 ist mit ihrem unteren Ende auf dem Schwungkörper 21 drehbar gelagert, und zwar genau in der Mitte. Das obere Ende der Koppelstange 23 ist auf dem Druckstück 9 drehbar gelagert. Schwungkörper 21 und Koppelstange 23 bilden ein passiv mitlaufendes Koppelgetriebe, das die Vor- und Rückbewegung der Treibstange 7 in eine Querbewegung des Schwungkörpers 21 umsetzt und umgekehrt. Die Bewegungsbahn 22 verläuft quer zur Stichachse.

In Fig. 3a hat die Vortriebsphase gerade begonnen.

In Fig. 3b hat die Treibstange 7 ihren Umkehrpunkt, welcher der maximalen Einstichtiefe entspricht, erreicht. Die Koppelstange 23 steht senkrecht. Das Koppelgetriebe befindet sich im Totpunkt. Die Druckfeder 10 ist maximal ausgelenkt. Der Schwungkörper 21 hat maximale Geschwindigkeit. Aufgrund seiner trägen Masse bewegt sich der Schwungkörper 21 in Richtung des Querpfeils quer zur Stichachse weiter. Dabei nähert sich das rechte Ende des Schwungkörpers einem Begrenzungselement, das auf dem Schiebeschlitten 5 fest montiert ist.

In Fig. 3c ist die schnelle Rückzugsphase gerade abgeschlossen. Die Druckfeder 10 ist bereits wieder ein Stück zusammengedrückt. Die Bewegungsenergie des Schwungkörpers 21 wurde auf die Treibstange 7 und die daran angekoppelte Lanzette (vgl. Fig. 1a) zurück verlagert. Der Stechantrieb wurde am Ende gestoppt, indem der Schwungkörper 21 mit seiner rechten Stirnseite an das Begrenzungselement 24 gestoßen ist.

Der Schwungkörper 21 ist hier aus ferromagnetischem Eisen hergestellt. Das Begrenzungselement 24 ist als Permanentmagnet ausgebildet, dessen Pole durch den Schwungkörper 21, der als magnetischere Anker wirkt, geschlossen werden. In der Endstellung werden also der Schwungkörper 21 und das Begrenzungselement 24 durch Magnetkraft aufeinander gehalten. Damit ist der Stechantrieb festgelegt.

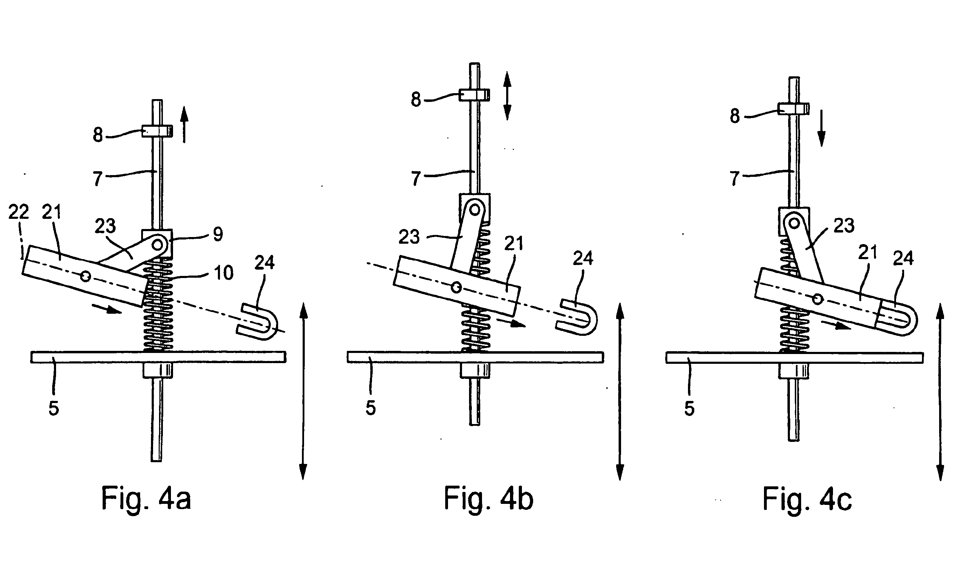

Die weitere Ausführungsform gemäß den Figuren 4a, 4b und 4c unterscheidet sich von der zuvor beschriebenen Ausführungsform nur dadurch, dass die Bewegungsbahn 22 des Schwungkörpers 21 nicht quer, sondern um ungefähr 15 Winkelgrade schräg gegenüber der Stichachse verläuft. Dadurch steht im oberen Totpunkt die Koppelstange 23 nicht senkrecht, sondern steht um den gleichen Winkel schräg nach links, wie in Fig. 4b zu sehen. Da sich der Mittelpunkt des Schwungkörpers 21, der gleichzeitig den Anlenkpunkt für die Koppelstange 23 bildet, auf einer schiefen Bahn nach unten bewegt, wird weniger kinetische Energie benötigt, um den Schwungkörper 21 zu beschleunigen.

### Verzeichnis der Bezugszeichen

- 1: Lanzette
- 2: Stechspitze
- 3: Kapillarkanal
- 4: Blutsammelraum
- 5: Schiebeschlitten
- 6: Gleitlager
- 7: Treibstange
- 8: Kupplung
- 9: Druckstück
- 10: Druckfeder
- 11: Schwungrad
- 11 a, 11 b: Schwungräder
- 12: Kurbelstange
- 12a, 12b: Kurbelstangen
- 13: Anschlagelement
- 14: Gegenanschlag
- 15: Querjoch
- 16a, 16b: Verzahnungen (an 11a, 11b)
- 17: Drehgelenk
- 18a, 18b: Kurbelzapfen
- 19: Spannanschlag (an 11a)
- 20: Spanngegenanschlag
- 21: Schwungkörper
- 22: Bewegungsbahn (von 21)
- 23: Koppelstange
- 24: Begrenzungselement

## Patentansprüche

1. Gerät zur Gewinnung einer Blutprobe, mit
einer Lanzette (1) die in einer kontrollierten Vor- und Rückbewegung in die Haut eines Körperteils eingestochen und sofort anschließend wieder ein Stück zurückgezogen wird, um das aus der Wunde austretende Blut aufzunehmen,
einem Stechantrieb umfassend eine an die Lanzette angekoppelte Treibstange (7) und eine in Richtung der Stichachse der Lanzette wirkende Antriebsfeder (10),
einer Steuereinrichtung zur Steuerung der Einstich- und Rückzugsbewegung der Lanzette gemäß einem vorgegebenen Bewegungsprofil umfassend zumindest eine schnelle Vortriebsphase, bei der die Lanzette bis zu einer vorgegebenen maximalen Einstichtiefe eingestochen wird, und eine schnelle Rückzugsphase, bei der die Lanzette bis zu einer vorgegebenen Reststichtiefe teilweise wieder zurückgezogen wird,
einer beweglich gelagerten Schwungmasse (11, 21) und einem Koppelglied (12, 23) **dadurch gekennzeichnet dass** die Schwungmasse an die Treibstange (7) derart ankoppelt, dass der Vortrieb der Lanzette (1) die Schwungmasse (11, 21) in Bewegung versetzt und nach Erreichen der maximalen Einstichtiefe die in der Schwungmasse gespeicherte Bewegungsenergie die Rückzugsbewegung der Lanzette (1) bewirkt.

2. Gerät nach Anspruch 1, **gekennzeichnet durch** ein Koppelgetriebe umfassend wenigstens einen, entlang einer linearen Bewegungsbahn bewegbaren Schwungkörper (21) und eine Koppelstange (23), deren eines Ende mit der Treibstange (7) verbunden ist und deren anderes Ende an dem Schwungkörper (21) beweglich gelagert ist, so dass der Vortrieb der Lanzette (1) den Schwungkörper (21) in eine translatorische Bewegung versetzt und nach Erreichen des Umkehrpunktes der Lanzette (1) die in dem Schwungkörper (21) gespeicherte Bewegungsenergie die Rückzugsbewegung der Lanzette (1) bewirkt.

3. Gerät nach Anspruch 2, **dadurch gekennzeichnet, dass** die Bewegungsbahn des Schwungkörpers (21) im Wesentlichen quer zur Stichachse der Lanzette (1) verläuft.

4. Gerät nach Anspruch 2, **dadurch gekennzeichnet, dass** die Bewegungsbahn des Schwungkörpers (21) eine Gerade ist, die schräg gegenüber der Stichachse der Lanzette (1) verläuft, vorzugsweise unter einem Winkel zwischen 5 und 45 Grad.

5. Gerät nach Anspruch 1, **dadurch gekennzeichnet, dass** die Bewegungsbahn der Schwungmasse gekrümmt ist, vorzugsweise kreisförmig.

6. Gerät nach Anspruch 1, **gekennzeichnet durch**
einen Kurbeltrieb umfassend wenigstens ein drehbar gelagertes Schwungrad (11) und eine Kurbelstange (12), deren eines Ende mit der Treibstange (7) verbunden ist und deren anderes Ende an dem Schwungrad (11) exzentrisch gelagert ist,
so dass der Vortrieb der Lanzette (1) das Schwungrad (11) in Rotation versetzt und nach Überwindung des oberen Totpunkts, der die maximale Einstichtiefe definiert, die in dem Schwungrad (11) gespeicherte Rotationsenergie die Rückzugsbewegung der Lanzette (1) bewirkt.

7. Gerät nach Anspruch 6, **dadurch gekennzeichnet, dass** die Drehachse des Schwungrads (11) quer zur Stichachse der Lanzette (1) angeordnet ist.

8. Gerät nach Anspruch 6 oder 7, **dadurch gekennzeichnet, dass** die Rückzugsbewegung der Lanzette (1) gegen die Spannkraft der Antriebsfeder (10) erfolgt.

9. Gerät nach einem der Ansprüche 6 bis 8, **dadurch gekennzeichnet, dass** die Drehachse des Schwungrads (11) mit seitlichem Abstand zur Bewegungsachse der Lanzette (1) angeordnet ist.

10. Gerät nach einem der Ansprüche 6 bis 9, **dadurch gekennzeichnet, dass**
die Antriebsfeder eine koaxial um die Treibstange (7) angeordnete Druckfeder (10) ist,
auf der Treibstange (7) ein Druckstück (9) vorgesehen ist, gegen das die Druckfeder (10) drückt,
die Kurbelstange (12) über das Druckstück (9) mit der Treibstange (7) verbunden ist.

11. Gerät nach einem der Ansprüche 6 bis 10, **dadurch gekennzeichnet, dass** das Schwungrad (11) ein mitbewegtes Anschlagelement (13) trägt, das bei einer vorgegebenen Winkelstellung auf einen festen Gegenanschlag (14) trifft, um die Drehbewegung des Schwungrads (11) zu stoppen und damit die Reststichtiefe zu definieren.

12. Gerät nach Anspruch 11, **dadurch gekennzeichnet, dass** das Anschlagelement (13) an dem Gegenanschlag (14) magnetisch haftet, um den Stechantrieb festzulegen.

13. Gerät nach einem der Ansprüche 6 bis 12, **dadurch gekennzeichnet, dass**
zwei Schwungräder (11a, 11b) einander gegenüberliegend zu beiden Seiten der Treibstange (7) angeordnet sind,
an der Treibstange (7) ein Querjoch (15) angeordnet ist,
die beiden gegenüberliegenden Enden des Querjochs (15) durch je eine Kurbelstange (12a, 12b) mit den Schwungrädern (11a, 11b) verbunden sind.

14. Gerät nach Anspruch 13, **dadurch gekennzeichnet, dass** die beiden Schwungräder (11a, 11b) miteinander synchronisiert sind.

15. Gerät nach Anspruch 14, **dadurch gekennzeichnet, dass** die beiden Schwungräder (11 a, 11b) an ihren Stirnseiten ineinander greifende Verzahnungen (16a, 16b) aufweisen.

16. Gerät nach einem der Ansprüche 13 bis 15, **dadurch gekennzeichnet, dass** das Querjoch (15) schwenkbeweglich gelagert ist.

17. Gerät nach einem der Ansprüche 6 bis 16, **dadurch gekennzeichnet, dass** der Stechantrieb und der Kurbelantrieb auf einem Schiebeschlitten (5) angeordnet sind, welcher parallel zur Bewegungsachse der Lanzette (1) verschieblich ist.

18. Gerät nach Anspruch 17, **dadurch gekennzeichnet, dass** auf zumindest einem Schwungrad (11a) des Kurbeltriebs ein Spannanschlag (19) sitzt, der infolge einer Verschiebung des Schiebeschlittens (5) entgegen der Vortriebsrichtung der Lanzette (1) auf einen Spanngegenanschlag (20) trifft, wodurch das Schwungrad (11a) zurückgedreht wird, um die Druckfeder (10) zu spannen.

## Claims

1. Device for acquiring a blood sample, comprising
a lancet (1), which, in a controlled forward and back movement, pierces into the skin of a body part and is subsequently immediately retracted again somewhat in order to receive the blood exiting from the wound,
a puncture drive comprising a drive rod (7) coupled to the lancet and a drive spring (10) acting in the direction of the piercing axis of the lancet,
a control unit for controlling the piercing and retraction movement of the lancet according to a predetermined movement profile comprising at least one rapid propulsion phase, in which the lancet pierces up to a predetermined maximum piercing depth, and a rapid retraction phase, in which the lancet is partially retracted again up to a predetermined residual piercing depth,
a movably mounted flywheel mass (11, 21), and a coupling element (12, 23),
**characterized in that**
the flywheel mass is coupled to the drive rod (7) in such a manner that the propulsion of the lancet (1) sets the flywheel mass (11, 21) into motion and, after reaching the maximum piercing depth, the movement energy stored in the flywheel mass causes the retraction movement of the lancet (1).

2. Device according to claim 1, **characterized in that** a coupling drive comprises at least one flywheel body (21), which is movable along a linear movement path, and a coupling rod (23), one end of which is connected to the drive rod (7) and the other end of which is mounted on the flywheel body (21) in such a manner that the propulsion of the lancet (1) sets the flywheel body (21) into a translational movement and, after reaching the reversal point of the lancet (1), the movement energy stored in the flywheel body (21) causes the retraction movement of the lancet (1).

3. Device according to claim 2, **characterized in that** the movement path of the flywheel body (21) runs essentially perpendicular to the piercing axis of the lancet (1).

4. Device according to claim 2, **characterized in that** the movement path of the flywheel body (21) is a straight line, which runs diagonally relative to the piercing axis of the lancet (1), preferably at an angle between 5 and 45°.

5. Device according to claim 1, **characterized in that** the movement path of the flywheel mass is curved, preferably circular.

6. Device according to claim 1, **characterized in that**
a crank drive comprises at least one flywheel (11), which is mounted so that it is rotatable, and a connecting rod (12), one end of which is connected to the drive rod (7) and the other end of which is mounted eccentrically on the flywheel (11),
so that the propulsion of the lancet (1) sets the flywheel (11) into rotation and, after overcoming top dead center, which defines the maximum piercing depth, the rotational energy stored in the flywheel (11) causes the retraction movement of the lancet (1).

7. Device according to claim 6, **characterized in that** the rotational axis of the flywheel (11) is located transversely to the piercing axis of the lancet (1).

8. Device according to claim 6 or 7, **characterized in that** the retraction movement of the lancet (1) occurs against the tension force of the drive spring (10).

9. Device according to any one of claims 6 to 8, **characterized in that** the rotational axis of the flywheel (11) is placed with lateral spacing to the movement axis of the lancet (1).

10. Device according to any one of claims 6 to 9, **characterized in that**
the drive spring is a compression spring (10) located coaxially around the drive rod (7),
a plunger (9) is provided on the drive rod (7), against which the compression spring (10) presses, and
the connecting rod (12) is connected via the plunger (9) to the drive rod (7).

11. Device according to any one of claims 6 to 10, **characterized in that** the flywheel (11) carries a driven stop element (13), which hits a fixed counter stop (14) at a predetermined angle, in order to stop the rotational movement of the flywheel (11) and thus define the residual piercing depth.

12. Device according to claim 11, **characterized in that** the stop element (13) adheres magnetically to the counter stop (14), in order to fix the puncture drive.

13. Device according to any one of claims 6 to 12, **characterized in that**
two flywheels (11a, 11b) are arranged opposite to one another on both sides of the drive rod (7),
a transverse yoke (15) is located on the drive rod (7), and
the two opposing ends of the transverse yoke (15) are each connected by a connecting rod (12a, 12b) to the flywheels (11a, 11b).

14. Device according to claim 13, **characterized in that** the two flywheels (11a, 11b) are synchronized with one another.

15. Device according to claim 14, **characterized in that** the two flywheels (11a, 11b) have gearings (16a, 16b), which engage with one another, on their front faces.

16. Device according to any one of claims 13 to 15, **characterized in that** the transverse yoke (15) is mounted so that it is pivotable.

17. Device according to any one of claims 6 to 16, **characterized in that** the puncture drive and the crank drive are arranged on a sliding carriage (5), which is displaceable parallel to the movement axis of the lancet (1).

18. Device according to claim 17, **characterized in that** a tension stop (19) is seated on at least one flywheel (11a) of the crank drive, which hits a tension counter stop (20) as a result of a displacement of the sliding carriage (5) opposite to the propulsion direction of the lancet (1), whereby the flywheel (11a) is rotated back in order to tension the compression spring (10).

## Revendications

1. Appareil pour obtenir un échantillon de sang, comportant
une lancette (1) qui, selon un mouvement contrôlé vers l'avant et vers l'arrière, pénètre dans la peau d'une partie du corps et se rétracte un peu aussitôt après afin de recueillir le sang sortant de la plaie,
un mécanisme de commande de piqûre comprenant une tige de commande (7) accouplée à la lancette et un ressort de commande (10) agissant en direction de l'axe de piqûre de la lancette,
un dispositif de commande du mouvement de pénétration et de rétraction de la lancette selon un profil de mouvement prédéfini comprenant au moins une phase de propulsion rapide consistant à faire pénétrer la lancette jusqu'à une profondeur de pénétration maximale prédéfinie et une phase de rétraction rapide consistant à rétracter la lancette partiellement jusqu'à une profondeur de pénétration restante prédéfinie,
une masse d'inertie (11, 21) montée mobile et un élément d'accouplement (12, 23),
**caractérisé en ce que** ladite masse inertie est accouplée à la tige de commande (7), de telle sorte que la propulsion de la lancette (1) entraîne en mouvement la masse d'inertie (11, 21) et, une fois la profondeur de pénétration maximale atteinte, l'énergie cinétique emmagasinée dans ladite masse d'inertie provoque le mouvement de rétraction de la lancette (1).

2. Appareil selon la revendication 1, **caractérisé par** un mécanisme d'accouplement comprenant au moins un corps d'inertie (21) susceptible de se déplacer selon une trajectoire linéaire et une barre d'accouplement (23) dont une extrémité est reliée à la tige de commande (7) et dont l'autre extrémité est montée mobile sur le corps d'inertie (21), de sorte que la propulsion de la lancette (1) entraîne le corps d'inertie (21) en un mouvement translatoire et, une fois le point d'inversion de la lancette (1) atteint, l'énergie cinétique emmagasinée dans le corps d'inertie (21) provoque le mouvement de rétraction de la lancette (1).

3. Appareil selon la revendication 2, **caractérisé en ce que** la trajectoire du corps d'inertie (21) s'étend essentiellement perpendiculairement à l'axe de piqûre de la lancette (1).

4. Appareil selon la revendication 2, **caractérisé en ce que** la trajectoire du corps d'inertie (21) est une droite qui s'étend obliquement par rapport à l'axe de piqûre de la lancette (1), de préférence sous un angle compris entre 5 et 45 degrés.

5. Appareil selon la revendication 1, **caractérisé en ce que** la trajectoire de la masse d'inertie est courbe, de préférence circulaire.

6. Appareil selon la revendication 1, **caractérisé par**
un mécanisme à manivelle comprenant au moins un volant d'inertie (11) monté mobile et une tige de manivelle (12) dont une extrémité est reliée à la tige de commande (7) et dont l'autre extrémité est montée excentriquement sur le volant d'inertie (11),
de sorte que la propulsion de la lancette (1) entraîne en rotation le volant d'inertie (11) et, une fois le point mort haut passé qui définit la profondeur de pénétration maximale, l'énergie de rotation emmagasinée dans le volant d'inertie (11) provoque le mouvement de rétraction de la lancette (1).

7. Appareil selon la revendication 6, **caractérisé en ce que** l'axe de rotation du volant d'inertie (11) est disposé perpendiculairement à l'axe de piqûre de la lancette (1).

8. Appareil selon la revendication 6 ou la revendication 7, **caractérisé en ce que** le mouvement de rétraction de la lancette (1) s'effectue à l'encontre de la force élastique du ressort de commande (10).

9. Appareil selon l'une des revendications 6 à 8, **caractérisé en ce que** l'axe de rotation du volant d'inertie (11) est disposé à distance latérale de l'axe de déplacement de la lancette (1).

10. Appareil selon l'une des revendications 6 à 9, **caractérisé en ce que**
le ressort de commande est un ressort de pression (10) disposé coaxialement autour de la tige de commande (7),
une pièce d'appui (9) est prévue sur la tige de commande (7), pièce contre laquelle appuie le ressort de pression (10),
la tige de manivelle (12) est reliée à la tige de commande (7) par l'intermédiaire de la pièce d'appui (9).

11. Appareil selon l'une des revendications 6 à 10, **caractérisé en ce que** le volant d'inertie (11) porte un élément de butée (13) mû en même temps et qui rencontre dans une position angulaire prédéfinie une contre-butée (14) fixe, afin de stopper la rotation du volant (11) et de définir ainsi la profondeur de pénétration restante.

12. Appareil selon la revendication 11, **caractérisé en ce que** l'élément de butée (13) tient magnétiquement à la contre-butée (14), afin de bloquer la commande de piqûre.

13. Appareil selon l'une des revendications 6 à 12, **caractérisé en ce que**
deux volants d'inertie (11a, 11b) sont disposés en regard l'un de l'autre de part et d'autre de la tige de commande (7),
une traverse (15) est disposée sur la tige de commande (7),
les deux extrémités opposées de la traverse (15) sont reliées aux volants d'inertie (11a, 11b), chacune par une tige de manivelle (12a, 12b).

14. Appareil selon la revendication 13, **caractérisé en ce que** les deux volants d'inertie (11 a, 11 b) sont synchronisés entre eux.

15. Appareil selon la revendication 14, **caractérisé en ce que** les deux volants d'inertie (11a, 11b) présentent sur leurs faces frontales des dentures (16a, 16b) s'engrenant l'une dans l'autre.

16. Appareil selon l'une des revendications 13 à 15, **caractérisé en ce que** la traverse (15) est montée pivotante.

17. Appareil selon l'une des revendications 6 à 16, **caractérisé en ce que** le mécanisme de commande de piqûre et le mécanisme à manivelle sont disposés sur un coulisseau (5), lequel peut se déplacer parallèlement à l'axe de déplacement de la lancette (1).

18. Appareil selon la revendication 17, **caractérisé en ce qu'**une butée de tension (19) est placée sur au moins un volant (11a) du mécanisme à manivelle, butée qui à la suite d'un déplacement du coulisseau (5) dans le sens contraire de la direction de propulsion de la lancette (1) rencontre une contre-butée de tension (20), induisant ainsi une rotation inverse du volant d'inertie (11a) afin de tendre le ressort de pression (10).
